# EUROPEAN PATENT APPLICATION

(11) **EP 3 747 498 A1**
(43) Date of publication of application: **09.12.2020**
(21) Application number: 18904057.9
(22) Date of filing: 01.02.2018
(51) Int. Cl.: A61M 25/09

(54) **GUIDE WIRE**

(71) Applicant: ASAHI INTECC CO., LTD., Seto-shi, Aichi 489-0071 (JP)
(72) Inventor: TSUGE Kenta, Aichi 489-0071 (JP)
(74) Representative: Winter, Brandl, Fürniss, Hübner, Röss, Kaiser, Polte - Partnerschaft mbB
(86) International application number: PCT/JP2018/003327
(87) International publication number: WO 2019/150520

(57) **Abstract**

[Problem to be Solved]

To provide a guidewire capable of ensuring flexibility of a distal portion of a guidewire and preventing a coating agent from peeling from a core shaft.

[Solution to Problem]

A guidewire 1 comprises a core shaft 3, a coil body 5 which a distal end of the coil body 5 is joined to the core shaft 3 at a position spaced proximally from a distal end of the core shaft 3, and a coating agent 7 covering an outer periphery of the core shaft 3 and the coil body 5, wherein the core shaft 3 includes a bulge portion 9 joined at a position spaced distally from the distal end of the coil body 5, the coating agent 7 covers the core shaft 3 and the coil body 5 from a distal side of the bulge portion 9 to a proximal side of a proximal end of the coil body 5 except for an interior of the coil body 5.

## Description

### TECHNICAL FIELD

The present invention relates to a medical guidewire.

### BACKGROUND ART

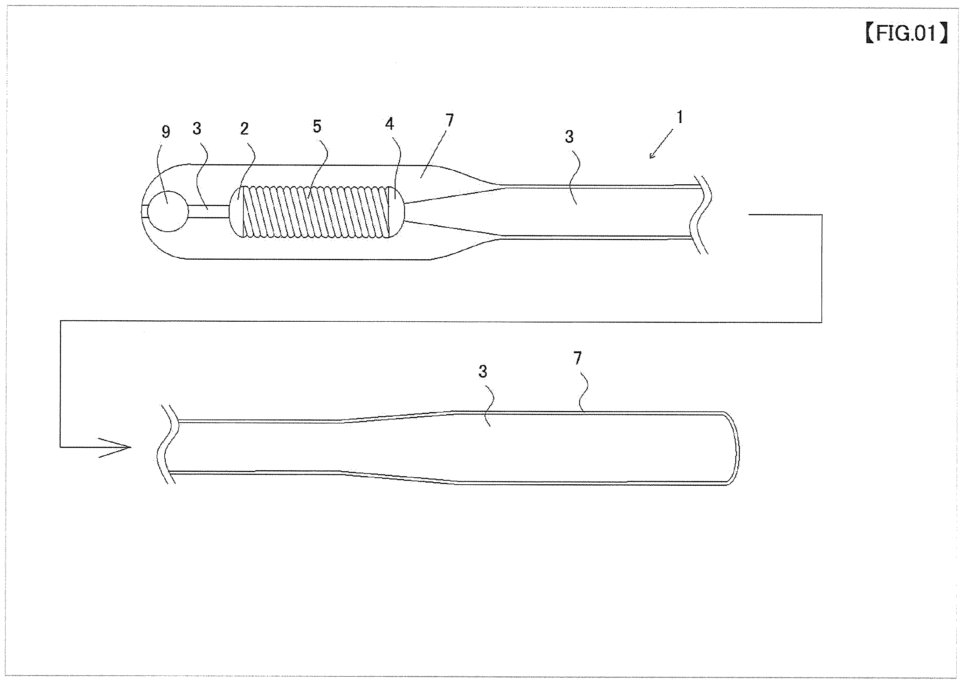

Conventionally, a variety of guidewires guiding a catheter or the like that is used by being inserted into tubular organs such as a blood vessel, a gastrointestinal tract and a ureter or body tissues for treatment or examination has been developed. For example, Patent Document 1 discloses a guidewire comprising a contrast portion at a distal portion of a core member, and a synthetic resin coating layer on an outer periphery of the core member and the contrast portion (see FIG.1 etc.). Further, a lubricious coating agent is applied to a surface of the synthetic resin coating layer described in Patent Document 1.

### PRIOR ART DOCUMENTS

### PATENT DOCUMENT

Patent Document 1 : Japanese Patent Application Publication No. 2004-41254 A

### SUMMARY OF THE INVENTION

### TECHNICAL PROBLEMS

As the guidewire described in Patent Document 1 includes a coil body being the contrast portion at the distal portion of the core member, and includes the synthetic resin coating layer on the outer periphery of the coil body, there is a problem that flexibility of a distal portion of the guidewire is impaired.

The synthetic resin coating layer and a lubricious coating agent formed on the surface of the synthetic resin coating layer (hereinafter, a synthetic resin coating layer and a lubricious coating agent are collectively referred to as "coating agent") are likely to be peeled from the core member in the guidewire described in Patent Document 1. Although the possibility of delamination has been slightly improved by the anchor effect due to an outer diameter of the contrast portion being larger than an outer diameter of the core member (hereinafter, referred to as "core shaft"), it was not sufficient yet.

The present invention has been made in response to forgoing problems of the convetional technique and is intended to provide a guidewire capable of ensuring flexibility of a distal portion of the guidewire and preventing the coating agent from peeling from the core shaft.

### SOLUTION FOR PROBLEMS

It is characterized in that, to solve the foregoing problems, a guidewire according to a first aspect of the present invention comprises a core shaft, a coil body which a distal end of the coil body is joined to the core shaft at a position spaced proximally from a distal end of the core shaft, and a coating agent covering an outer periphery of the core shaft and the coil body, wherein the core shaft includes a bulge portion joined at a position spaced distally from the distal end of the coil body, the coating agent covers the core shaft and the coil body from a distal side of a distal end of the bulge portion to a proximal side of a proximal end of the core shaft except for an interior of the coil body.

A second aspect of the present invention is characterized in that, in the guidewire according to the first aspect, further comprises a stranded wire joined to a distal portion of the core shaft,-including a plurality of wires and extending distally from the distal end of the core shaft, wherein the coil body extends distally from the distal end of the core shaft halfway through the stranded wire, the coating agent covers an outer periphery of the stranded wire, the core shaft and the coil body, the bulge portion is joined to the stranded wire at a position spaced distally from a distal end of the coil body, the coating agent covers the stranded wire, the core shaft and the coil body from the distal side of the distal end of the bulge portion to the proximal side of the proximal end of the core shaft except for the interior of the coil body.

Furthermore, a third aspect of the present invention is characterized in that, in the guidewire according to the second aspect of the present invention, the coating agent penetrates between a plurality of wires included in the stranded wire.

### ADVANTAGEOUS EFFECTS OF INVENTION

According to the guidewire of the first aspect of the present invention, as a guidewire comprises a core shaft, a coil body which a distal end of the coil body is joined to the core shaft at a position spaced proximally from a distal end of the core shaft, and a coating agent covering an outer periphery of the core shaft and the coil body, wherein the core shaft includes a bulge portion joined at a position spaced distally from the distal end of the coil body, the coating agent covers the core shaft and the coil body from a distal side of a distal end of the bulge portion to a proximal side of a proximal end of the core shaft except for an interior of the coil body, it is capable of ensuring flexibility of the coil body and a distal portion of the guidewire and preventing the coating agent from peeling from the core shaft and the coil body by the bulge portion and the coil body.

Further, according to the guidewire of the second aspect of the present invention, in the guidewire of the first aspect, as the guidewire further comprises a stranded wire joined to a distal portion of the core shaft, inclulding a plurality of wires and extending distally from the distal end of the core shaft, wherein the coil body extends distally from the distal end of the core shaft halfway through the stranded wire, the coating agent covers an outer periphery of the stranded wire, the core shaft and the coil body, the bulge portion is joined to the stranded wire at a position spaced distally from a distal end of the coil body, the coating agent covers the stranded wire, the core shaft and the coil body from the distal side of the distal end of the bulge portion to the proximal side of the proximal end of the core shaft except for the interior of the coil body, it is capable of ensuring more flexibility of the coil body, the stranded wire and a distal portion of the guidewire and further preventing the coating agent from peeling from the core shaft, the stranded wire and the coil body by the bulge portion and the coil body.

Furthermore, according to the guidewire of the third aspect of the present invention, in the guidewire according to the second aspect of the present invention, as the coating agent penetrates between a plurality of wires included in the stranded wire, it is capable of further preventing the coating agent from peeling from the stranded wire.

### BRIEF DESCRIPTION OF THE DRAWINGS

FIG. 1 is a schematic view of a guidewire of a first embodiment of the present invention.
FIG. 2 is a longitudinal sectional view of the guidewire of the first embodiment.
FIG.3 is a schematic diagram of a guidewire of a second embodiment.
FIG.4 is a longitudinal sectional view of the guidewire of the second embodiment.
FIG.5 is a longitudinal sectional view of a distal portin of a guidewire of a third embodiment.
FIG.6 is a longitudinal sectional view near a stranded wire included in the guidewire of the third embodiment.

### DESCRIPTION OF EMBODIMENTS

Hereinafter, embodiments of the present invention will be described with reference to the drawings.

### (First Embodiment)

First of all, a first embodiment of the present invention will be described.

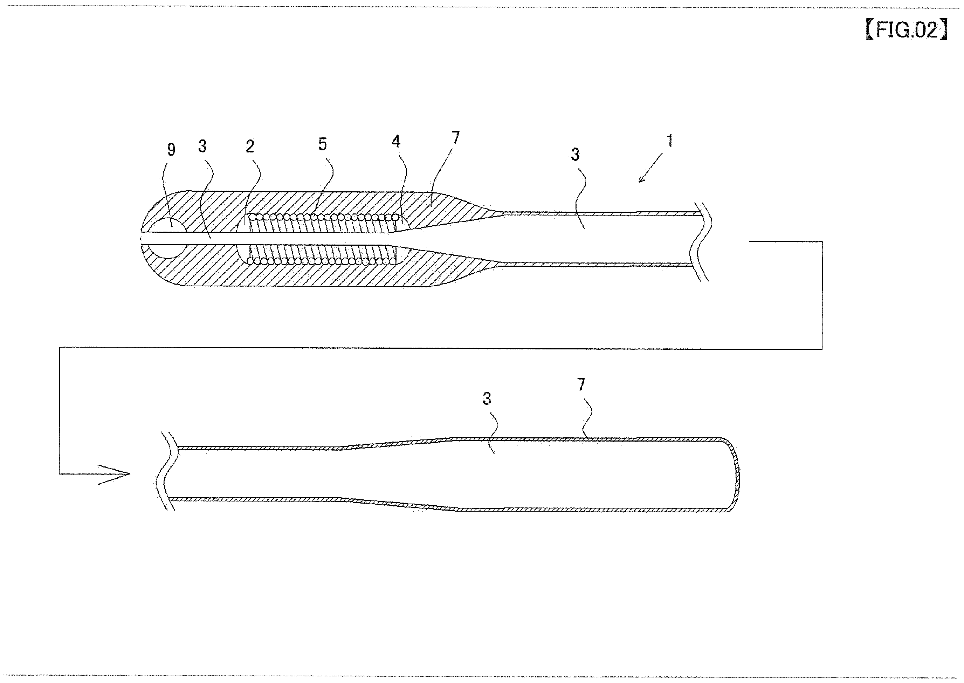

FIG. 1 is a schematic view of a guidewire of a first embodiment of the present invention, FIG. 2 is a longitudinal sectional view of the guidewire of the first embodiment.

As shown in FIG. 1, a guidewire 1 of this embodiment includes a core shaft 3, a coil body 5 joined to the core shaft 3 at a position spaced proximally from a distal end of the core shaft 3, a bulge portion 9 joined to the core shaft 3 at a position spaced distally from a distal end of the coil body 5, and a coating agent 7 covering an outer periphery of the core shaft 3, the coil body 5 and the bulge portion 9.

The core shaft 3 is a rod-like member of circular cross-section which is reduced in diameter toward a distal end from a proximal end of the core shaft 3, is an elongate flexible member. Material of the core shaft 3 is not particularly limited as long as it is biocompatible material such as stainless steel, Ni-Ti-based alloys, cobalt based alloys or the like. Stainless steel is used in the present embodiment.

The coil body 5 is joined to the core shaft at a position spaced proximally from a distal end of the core shaft 3. Specifically, a distal end of the coil body 5 is joined to the core shaft 3 by brazing material 2, a proximal end of the coil body 5 is joined to the core shaft 3 by brazing material 4.

Incidentally, the brazing material 2 and the brazing material 4 is not particularly limited as long as it is biocompatible brazing material such as gold-tin brazing material, silver-tin brazing material or the like. Silver-tin brazing material is used in the present embodiment. The coil body 5 is a cylindrical hollow coil which is formed by winding a single metal wire or a plurality of metal wires, an outer diameter of the coil body 5 is larger than an outer diameter of the core shaft 3 in the position in which the coil body 5 is joined.

Material of the metal wire forming the coil body 5 is not particularly limited as long as it is biocompatible material such as stainless steel, tangsten, Ni-Ti-based alloys alloys or the like. Stainless steel is used in the present embodiment.

The bulge portion 9 is joined to the distal end of the core shaft 3 at a position spaced distally from the distal end of the coil body 5. An outer diameter of the bulge portion 9 is greater than an outer diameter of the core shaft 3 at a position which the bulge portion 9 is joined to the core shaft 3. Material used for the bulge portion 9 is generally a brazing material similar to the brazing material 2 and the brazing material 4 joining the coil body 5 to the core shaft 3.

An area of the coating agent 7 is illustrated by hatching in FIG. 2, as shown in FIG. 2, the coating agent 7 covers the core shaft 3, the coil body 5 and the bulge portion 9 from a distal end of the guidewire 1 being a distal side of the distal end of the bulge portion 9 to the proximal side of the proximal end of the core shaft 5 except for the interior of the coil body 5.

It is to ensure the flexibility of the distal end of the guidewire 1 not to allow the coating agent 7 to penetrate inside the coil body 5.

Incidentally, the coating agent 7 of the present embodiment covers the core shaft 3 to the proximal end of the core shaft 3 in a proximal side of the proximal end of the coil body 5 in order to reduce frictional resistance of the guidewire 1.

The coating agent 7 is preferably formed by low friction material, e.g. polyvinyl alcohol, polyvinyl pyrrolidone, polyethylene glycol, polyacrylamide, polyacrylic acid, sodium polyacrylate, poly (2-hydroxyethyl methacrylate), maleic anhydride copolymer, ethylene-vinyl alcohol copolymer, 2-methacryloyloxyethyl phosphorylcholine or a copolymer thereof, (2-hydroxyethyl methacrylate) - styrene block copolymer, various synthetic polypeptides, collagen, hyaluronic acid, cellulose-based polymer, and mixtures thereof.

Further, the coating agent 7 may be made of different materials depending on a position of the guidewire 1. For example, material of a distal side region from a middle position of the coil body 5 may be a coating agent 7 made of different materials from material of a proximal side region therefrom.

According to the guidewire 1 of this embodiment, as a guidewire 1 comprises a core shaft 3, a coil body 5 which a distal end of the coil body 5 is joined to the core shaft 3 at a position spaced proximally from a distal end of the core shaft 3, and a coating agent 7 covering an outer periphery of the core shaft 3 and the coil body 5, wherein the core shaft 3 includes a bulge portion 9 joined at a position spaced distally from the distal end of the coil body 5, the coating agent 7 covers the core shaft 3 and the coil body 5 from a distal side of a distal end of the bulge portion 9 to a proximal side of a proximal end of the core shaft 5 except for an interior of the coil body 5, it is capable of ensuring flexibility of the coil body 5 and a distal portion of the guidewire 1 and preventing the coating agent 7 from peeling from the core shaft 3 and the coil body 5 by the bulge portion 9 and the coil body 5.

The bulge portion 9 may be joined to the distal end of the core shaft 3 so that the distal end of the core shaft 3 does not protrude from the bulge portion 9. According to this configuration, it is capable of preventing the distal end of the core shaft 3 from protruding from the coating agent 7 and damaging to the body tissue.

### (Second Embodiment)

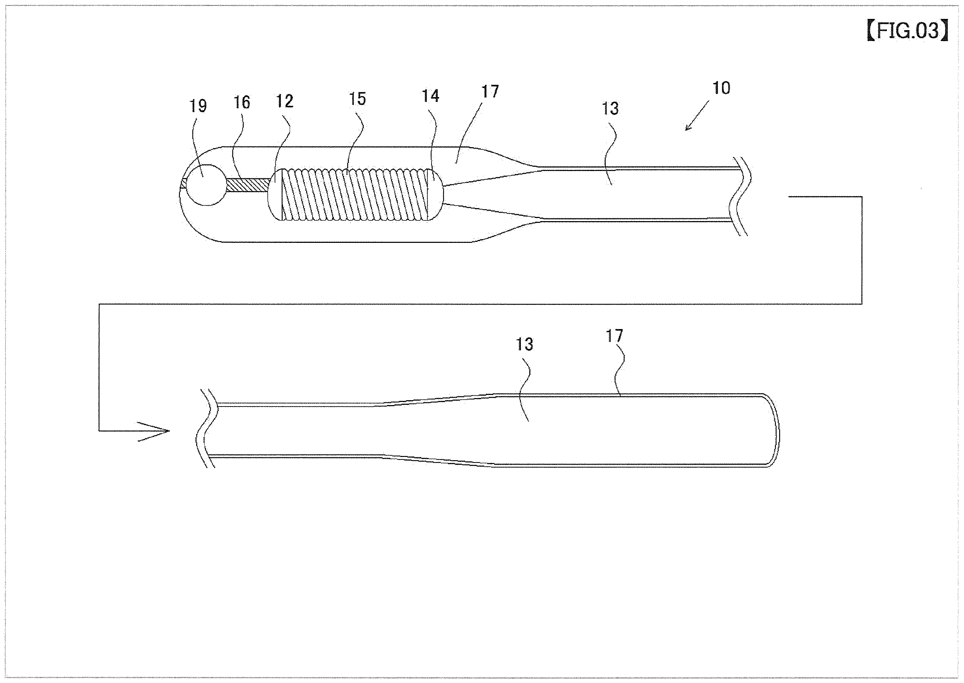

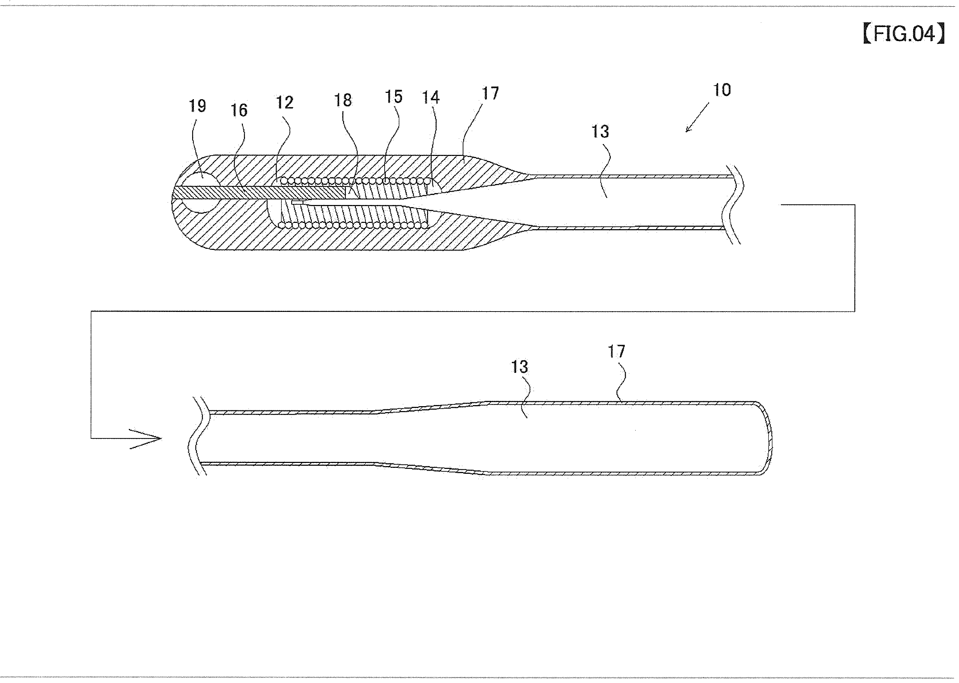

Next, a second embodiment of the present invention will be described. FIG.3 is a schematic diagram of a guidewire of a second embodiment, FIG.4 is a longitudinal sectional view of the guidewire of the second embodiment.

As shown in FIG. 3, a guidewire 10 of this embodiment includes a core shaft 13, a stranded wire 16 joined to a distal portion of the core shaft 13 so as to be located on a distal side of the core shaft 13 and including a plurality of wires, a coil body 15 having a distal end joined to the stranded wire 16 at a position spaced proximally from a distal end of the stranded wire 16 and a proximal end joined to the core shaft 13, a coating agent 17 covering an outer periphery of the core shaft 13, the stranded wire 16 and the coil body 15, and a bulge portion 19 joined to the stranded wire 16 at a position spaced distally from a distal end of the coil body 5.

The core shaft 13 is a rod-like member of circular cross-section which is reduced in diameter toward a distal end from a proximal end of the core shaft 13, is an elongate flexible member similarly to the core shaft 3. Material of the core shaft 13 is same as that of the core shaft 3.

As shown in FIG. 4, the stranded wire 16 includes a proximal end joined to a distal portion of the core shaft 13 by a brazing material 18 inside the coil body 15, and extends distally from a distal end of the core shaft 13 and a distal end of the coil body 15.

Incidentally, the stranded wire 16 may be joined to the coil body 15 not only the core shaft 13 by brazing material 18.

The coil body 15 includes a distal end joined to the stranded wire 16 and a proximal end joined to the core shaft 13. Specifically, the distal end of the coil body 15 is joined to the stranded wire 16 by a brazing material 12, and the proximal end of the coil body 15 is joined to the core shaft 13 by a brazing material 14.

Incidentally, the brazing material 12, the brazing material 14 and the brazing material 18 may use a material similar to that of the brazing material 2 and the brazing material 4 of the first embodiment. Also, the coil 15 is similar to the coil body 5 of the first embodiment, an outer diameter of the coil body 15 is larger than an outer diameter of the core shaft 13 in the position in which the coil body 15 is joined.

The bulge portion 19 is joined to the stranded wire 16 at a position spaced distally from the distal end of the core shaft 13 and the distal end of the coil body 15. An outer diameter of the bulge portion 19 is greater than an outer diameter of the core shaft 13 at a position which the bulge portion 19 is joined to the core shaft 13. Incidentally, material of the bulge portion 19 is also similar to that of the bulge portion 9 of the first embodiment.

An area of the coating agent 17 is illustrated by hatching in FIG. 4, as shown in FIG. 4, the coating agent 17 covers the core shaft 13, the coil body 15 and the bulge portion 19 from a distal end of the guidewire 10 being a distal side of the distal end of the bulge portion 19 to the proximal side of the proximal end of the coil body 15 except for the interior of the coil body 15.

It is to ensure the flexibility of the distal end of the guidewire 10 not to allow the coating agent 17 to penetrate inside the coil body 15.

Incidentally, the coating agent 17 of the present embodiment covers the core shaft 13 to the proximal end of the core shaft 13 even in the proximal side of the proximal end of the coil body 15. Material of the coating agent 17 is similar to that of the coating agent 7 of the first embodiment.

According to the guidewire 10 of the present embodiment, as a guidewire 10 comprises a core shaft 13, a stranded wire 16 joined to a distal portion of the core shaft 13 so as to be located on a distal side of the core shaft 13 and including a plurality of wires, a coil body 15 having a distal end joined to the stranded wire 16 at a position spaced proximally from a distal end of the stranded wire 16 and a proximal end joined to the core shaft 13, a coating agent 17 covering an outer periphery of the stranded wire 16, the core shaft 13 and the coil body 15, and a bulge portion 19 joined to the stranded wire 16 at a position spaced distally from a distal end of the coil body 5 wherein the coating agent 17 covers the core shaft 13 and the coil body 15 from a distal side the bulge portion 19 to a proximal side of the proximal end of the coil body 15 except for the interior of the coil body 15, it is capable of ensuring flexibility of the coil body 15 and a distal portion of the guidewire 10 and preventing the coating agent 17 from peeling from the staranded wire 16, the core shaft 13 and the coil body 15 by the bulge portion 19 and the coil body 15.

The bulge portion 19 may be joined to the distal end of the stranded wire 16 so that the distal end of the stranded wire 16 does not protrude from the bulge portion 19. According to this configuration, it is capable of preventing the distal end of the stranded wire 16 from protruding from the coating agent 17 and damaging to the body tissue.

### (Third Embodiment)

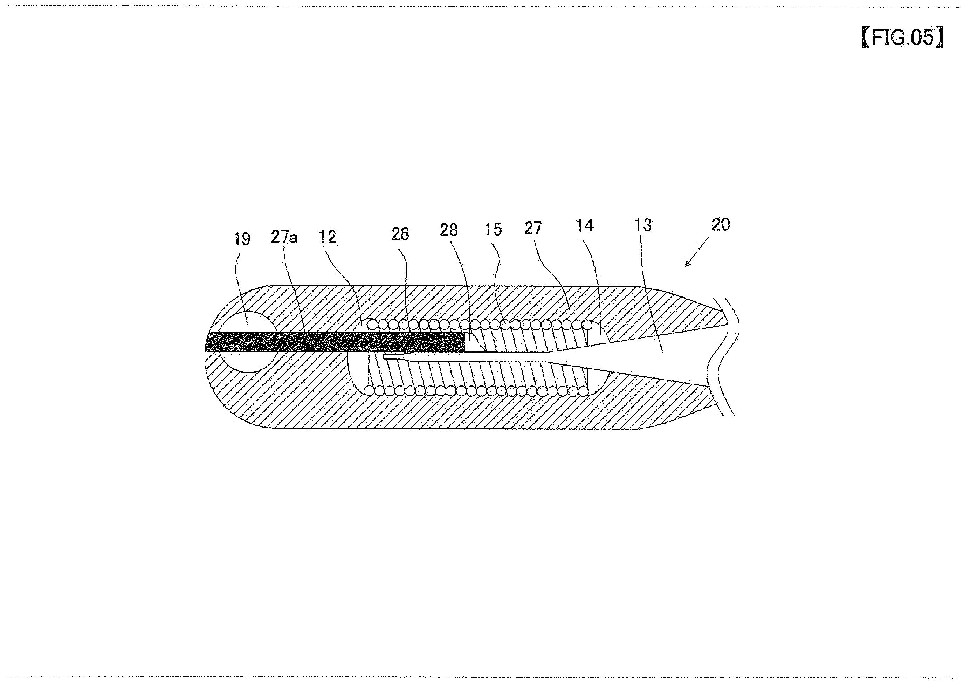

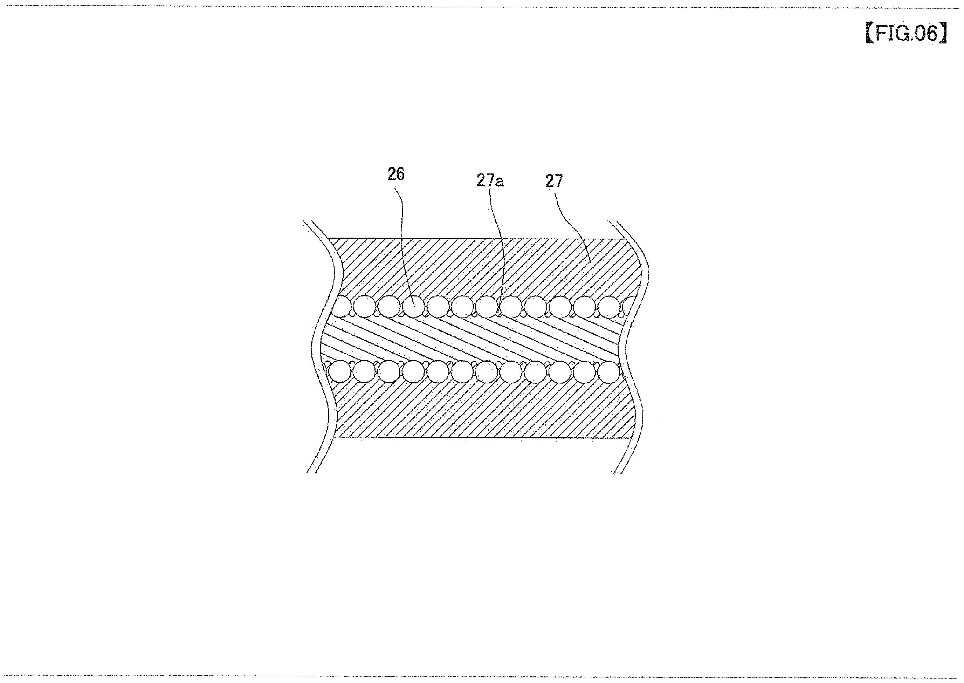

Next, a third embodiment of the present invention will be described. FIG.5 is a longitudinal sectional view of a distal portin of a guidewire of a third embodiment, FIG.6 is a longitudinal sectional view near a stranded wire included in the guidewire of the third embodiment.

Incidentally, portions common to the second embodiment will be denoted by the same reference numerals, descriptions of the portions will be omitted.

The guidewire 20 of the present embodiment is different from the guidewire 10 of the second embodiment in connecting state between the stranded wire and the coating agent. That is, a coating agent 27 penetrates between a plurality of wires included in a stranded wire 26 to form a protruding portion 27a that protrudes from a gap between a plurality of wires included in a stranded wire 26.

The guidewire of this embodiment increases bonding strength between the coating agent 27 and the stranded wire 26 by forming the protruding portion 27a. Further, as the protruding portion 27a of this embodiment has an outer diameter greater than a spacing between wires in the lower part of the wires included in the stranded wire 26, it is capable of further preventing the coating agent 27 from peeling from the staranded wire 26.

As shown in FIG. 5, the stranded wire 26 includes a proximal end joined to a distal portion of the core shaft 13 by a brazing material 28 inside the coil body 15, and extends distally from a distal end of the core shaft 13 and a distal end of the coil body 15. Incidentally, the brazing material 28 may use a material similar to that of the brazing material 2 and the brazing material 4 of the first embodiment.

According to the guidewire 20 of the present embodiment, as the coating agent 27 penetrates between a plurality of wires included in the stranded wire 26, it is capable of further preventing the coating agent 27 from peeling from the stranded wire 26.

The bulge portion 19 may be joined to the distal end of the stranded wire 26 so that the distal end of the stranded wire 26 does not protrude from the bulge portion 19. According to this configuration, it is capable of preventing the distal end of the stranded wire 26 from protruding from the coating agent 27 and damaging to the body tissue.

### DESCRIPTION OF THE CODE

- 1, 10, 20: guidewire
- 2, 4, 12, 14, 18, 28: brazing material
- 3, 13: core shaft
- 5, 15: coil body
- 7, 17, 27: coating agent
- 9, 19: bulge portion
- 16, 26: stranded wire
- 27a: protrusion

## Claims

1. A guidewire, comprising:
a core shaft;
a coil body which a distal end of the coil body is joined to the core shaft at a position spaced proximally from a distal end of the core shaft; and
a coating agent covering an outer periphery of the core shaft and the coil body,
wherein the core shaft includes a bulge portion joined at a position spaced distally from the distal end of the coil body,
the coating agent covers the core shaft and the coil body from a distal side of a distal end of the bulge portion to a proximal side of a proximal end of the core shaft except for an interior of the coil body.

2. A guidewire according to Claim 1, further comprising:
a stranded wire joined to a distal portion of the core shaft, including a plurality of wires and extending distally from the distal end of the core shaft,
wherein the coil body extends distally from the distal end of the core shaft halfway through the stranded wire,
the coating agent covers an outer periphery of the stranded wire, the core shaft and the coil body,
the bulge portion is joined to the stranded wire at a position spaced distally from a distal end of the coil body,
the coating agent covers the stranded wire, the core shaft and the coil body from the distal side of the distal end of the bulge portion to the proximal side of the proximal end of the core shaft except for the interior of the coil body.

3. A guidewire according to Claim 2,
wherein the coating agent penetrates between a plurality of wires included in the stranded wire.
